**Europäisches Patentamt**

**European Patent Office**

**Office européen des brevets**

(19)

(11) Veröffentlichungsnummer : **0 510 566 B1**

## (12) EUROPÄISCHE PATENTSCHRIFT

(45) Veröffentlichungstag der Patentschrift :
**03.08.94 Patentblatt 94/31**

(21) Anmeldenummer : **92106755.9**

(22) Anmeldetag : **21.04.92**

(51) Int. Cl.⁵ : **B01J 37/00,** B01J 27/199,
C07C 51/25, C07C 51/23,
C07C 51/377, C07C 57/04

(54) Molybdän, Phosphor und Vanadium enthaltende Oxidationskatalysatoren mit aus verbrennbaren Fasermaterialien geformten Kanälen.

(30) Priorität : **25.04.91 DE 4113423**

(43) Veröffentlichungstag der Anmeldung :
**28.10.92 Patentblatt 92/44**

(45) Bekanntmachung des Hinweises auf die
Patenterteilung :
**03.08.94 Patentblatt 94/31**

(84) Benannte Vertragsstaaten :
**AT BE CH DE DK ES FR GB IT LI NL SE**

(56) Entgegenhaltungen :
**EP-A- 0 284 947**

(56) Entgegenhaltungen :
**DE-A- 1 803 773**
**DE-A- 1 805 386**
**US-A- 4 092 269**
**US-A- 4 329 520**

(73) Patentinhaber : **RÖHM GMBH**
**Kirschenallee**
**D-64293 Darmstadt (DE)**

(72) Erfinder : **Bielmeier, Ernst, Dr.**
**Frankfurter Strasse 12**
**W-6103 Griesheim (DE)**
Erfinder : **Haeberle, Thomas, Dr.**
**Heinrich-Fey-Strasse 18a**
**W-6141 Einhausen (DE)**

EP 0 510 566 B1

**Beschreibung**

Die vorliegende Erfindung betrifft verbesserte Katalysatoren für die Gasphasenoxidation organischer Verbindungen und ein Verfahren zu deren Herstellung. Insbesondere dazu betrifft dies oxidische Mo, P und V enthaltende Katalysatoren für die Herstellung von ungesättigten Aldehyden und ungesättigten Carbonsäuren, vor allem solche für die Herstellung von Methacrolein und Methacrylsäure.

Katalysatoren oxidischer Art, die Molybdän, Phosphor und Vanadium als wesentliche Elemente enthalten, sind vor allem als Heteropolysäurekatalysatoren für die Durchführung selektiver Oxidationen zur Herstellung ungesättigter aliphatischer Verbindungen, wie Acrolein, Methacrolein und Acrylsäure sowie Methacrylsäure bekannt. So können nach der DE-A 27 22 375 z.B. $H_5PMo_{10}V_2O_{40}$-Heteropolysäure-haltige Katalysatoren sowohl für die Oxidation von Methacrolein zu Methacrylsäure als auch für die Oxidehydrierung von Isobuttersäure oder deren Ester zu Methacrylsäure oder deren Ester eingesetzt werden. Nach dem EP-B 0 005 769 werden Katalysatoren der Formel $Mo_aV_bP_cX_dO_e$, die Heteropolysäurestruktur haben, zur Oxidation von Isobutylen und/oder tert.-Butanol zu Methacrolein und Methacrylsäure verwendet. Ein Verfahren zur Oxidation von Methacrolein zu Methacrylsäure an Katalysatoren der gleichen Art ist in der DE-A 30 10 434 beschrieben.

Die Heteropolysäure der Formel $H_8PMo_{10}VO_{39}$ bzw. deren Anhydrid $PMo_{10}VO_{35}$ ist nach DE-A 35 08 649 als Katalysator in Oxidationsreaktionen, wie Oxidationen der Olefine Propylen und Isobutylen zu den entsprechenden ungesättigten Aldehyden Acrolein und Methacrolein und auch zu deren Weiteroxidation zu Acrylsäure und Methacrylsäure, und insbesondere in Oxidehydrierungsreaktionen von Isobuttersäure oder ihrer Ester zu Methacrylsäure oder ihrer Ester geeignet. Nach der EP-B 0 113 084 sind Kupferderivate dieses Heteropolysäureanhydrids, z.B. $Cu_{0,2}PMo_{10}VO_{35,2}$ und auch solche von anderen Heteropolysäuren, wie der $H_5PMo_{10}V_2O_{40}$-Heteropolysäure, sehr selektive Katalysatoren bei der Oxidehydrierung von Isobuttersäure zu Methacrylsäure.

In der DE-A 37 10 784 ist die Herstellung von Molybdän-Heteropolysäure-Oxidationskatalysatoren beschriebener Art angegeben, wobei wasserlösliche, praktisch nichtflüchtige organische Verbindungen, insbesondere Polymere, mitverwendet werden, die dann bei 150 bis 400 Grad C in Gegenwart von Sauerstoff calciniert werden. So hergestellte Katalysatoren zeichnen sich bei der Oxidehydrierung von Isobuttersäure zu Methacrylsäure durch ein verbessertes Langzeitverhalten aus. Schließlich wird in der EP-A 0 376 117 ein Verfahren zur Herstellung von Methacrylsäure durch Oxidation von Methacrolein an oxidischen Katalysatoren der Formel $Mo_{12}P_aV_bCs_cAs_dCu_eX_fY_gO_x$ vorgeschlagen, denen bei ihrer Formgebung neben bekannten Kohlenstoff-haltigen Verbindungen als Gleitmittel noch Formhilfsmittel und Verstärkungsmittel wie Microfasern aus anorganischen Materialien wie beispielsweise Glas oder Asbest zugesetzt werden können, und die bei Temperaturen von 180 bis 480 Grad C, gegebenenfalls in Luftatmosphäre, calciniert sind.

Katalysatoren für selektive Oxidation haben im allgemeinen eine kleine innere Oberfläche, d.h. das Katalysatorkorn ist mit relativ wenigen Poren versehen, oder aber sie werden mit porösen Trägern mit weiträumigen Poren im Innern des Trägermaterials hergestellt, in die das katalytische Material dann eingebettet ist. Solche Träger sind für einen Diffusionsstrom zum Materie- und Energietransport an das katalytisch aktive Material gut durchlässig. Kombinationen von Molybdän-, Vanadium-, Phosphor- und Sauerstoff-haltigen Stoffen und Trägern mit einer Porosität von 10 bis 80 % und einer inneren Oberfläche unter 1 m²/g sind als Katalysatoren für die Oxidehydrierung von Isobuttersäure zu Methacrylsäure in der DE-A 31 45 091 beschrieben.

Die Wirksamkeit von Katalysatoren in Gasphasenoxidations-Verfahren hängt von einer Reihe von Maßnahmen ab. Dazu zählen neben den oben diskutierten Unterschieden, wie den Zusammensetzungen der katalytisch aktiven Spezies, den Zusammensetzungen der umzusetzenden Gasgemische oder den Umsetzungsbedingungen, insbesondere der Reaktionstemperatur, auch eine optimale Porenmenge mit optimaler Porengrößenverteilung im katalytisch aktiven Material selbst, wodurch Ausbeute- und Selektivitätswerte des Wertproduktes und auch die Lebensdauer bestimmt werden.

Bei der Konfektionierung der Katalysatoren, d.h. bei deren Formgebung, werden die aktiven Bestandteile, gegebenenfalls in Gegenwart von Trägermaterialien bzw. inerten, anorganischen Verdünnungsmitteln, zu Pellets komprimiert oder zu stabartigen Teilchen extrudiert, wodurch ein Katalysator entsteht, der infolge weitgehendst fehlender Porosität im wesentlichen nur durch das an der Oberfläche vorhandene Material katalytisch wirkt, was sich insbesondere in niedrigen Raum-Zeit-Ausbeuten und in einer niedrigen Katalysatorlebensdauer niederschlägt. Andererseits bringen Katalysatoren, die mit Trägern von hoher innerer Oberfläche hergestellt sind, wie oben diskutiert und in der DE-A 31 45 091 für die Oxidehydrierung von Isobuttersäure zu Methacrylsäure gezeigt, in Abhängigkeit vom Anstieg der inneren Oberfläche, abfallende Ausbeute- und Selektivitätswerte.

Der Erfindung liegt die Aufgabe zugrunde, verbesserte Oxidationskatalysatoren, insbesondere solche oxidischer Art, die Molybdän, Phosphor und Vanadium als wesentliche Elemente enthalten, und die vor allem für die Herstellung ungesättigter Aldehyde und ungesättigter Carbonsäuren mit 3 und 4 Kohlenstoffatomen brauchbar sind, in Teilchenform mit guter Festigkeit zu schaffen, und die infolge einer poröseren Beschaffenheit, höhere katalytische Aktivitäten haben, die sich in höheren Raum-Zeit-Ausbeuten, als auch in höheren Selektivitäten zeigen.

Diese Aufgabe wird nach der Erfindung dadurch gelöst, daß zur Konfektionierung der Katalysatoren durch Formgebung, Mischungen von Katalysatorkomponenten und von Kohlenstoffhaltigen und organischen Fasern, die in Mengen von 0,5 bis 5 Gew.-% bezogen auf das katalytisch wirkende Material vorhanden sind, verwendet werden, und wonach in den Katalysatorformteilchen nach deren Calcinierung im Temperaturbereich von 100 bis 380 Grad C, Transportkanäle vorhanden sind.

Die Erfindung betrifft daher:

Gasphasenoxidationskatalysatoren, bestehend aus dem katalytisch aktiven Material, das Molybdän, Phosphor und Vanadium als wesentliche Elemente in oxidischer Form enthält, und gegebenenfalls noch aus weitgehend chemisch inerten anorganischen Bindemitteln, Verdünnungsmitteln bzw. Trägern, sowie im wesentlichen gleichförmig verteilten Kanälen,
dadurch gekennzeichnet,
daß bei der Herstellung der Katalysatoren mit Stoffen der chemischen Zusammensetzung $H_aM_bP_cMo_dV_eO_f$, wobei bedeuten

M mindestens ein Metallion von Li, Na, K, Rb, Cs, Mg, Ca, Zn, Al, Ce, Ti, Zr, Sn, Sb, As, Bi, Cr, Mn, Fe, Co, Ni oder Cu

a = 0 bis 8

b = 0 bis 6

c = 1 bis 2

d = 9 bis 12

e = 0,2 bis 3

f = Atomzahl, die sich zur stöchiometrischen Absättigung der übrigen Komponenten aus deren Wertigkeiten und Anteilen ergibt

als dem katalytisch aktiven Material oder als dessen Vorläufer und gegebenenfalls aus den inerten Zusatzstoffen, vor der Formgebung noch 0,5 bis 5 Gew.-%, bezogen auf das katalytisch wirkende Material, Kohlenstoff- bzw. organische Fasern mit einem Durchmesser von 1 bis 100 um und einer Länge von 1 bis 10 mm zugemischt werden, und die nach der Formgebung zur Bildung der Kanäle, durch Calcinieren der Formlinge bei Temperaturen im Bereich von 100 bis 380 Grad C in Gegenwart von Sauerstoff wieder daraus entfernt werden.

Die Stoffe der chemischen Zusammensetzung $H_aM_bP_cMo_dV_eO_f$ mit den oben angegebenen Bedeutungen und mit herausragend guten katalytischen Eigenschaften, sind vor allem Heteropolysäuren des Molybdäns mit Phosphor als Zentralatom, wie z.B. $H_5PMo_{10}V_2O_{40}$, $H_4PMo_{11}VO_{40}$ oder deren Salze.

So hergestellte Katalysatoren sind, wie dies für Oxidationskatalysatoren notwendig und üblich, oberflächenarme Gebilde, d.h. ihre inneren Oberflächen liegen im Bereich von ca. 0,1 bis 5 $m^2$/g vor allem im Bereich von 0,5 bis 2,5 $m^2$/g, d.h. etwa in der Größenordnung von 1 $m^2$/g. Dies bedeutet, daß durch die Bildung der für den verbesserten Materie- und Energietransport und damit der Verminderung der Diffusionshemmung notwendigen Kanäle, die innere Oberfläche des Katalysators durch die neuen Verfahrensmerkmale zur Katalysatorherstellung nicht nennenswert erhöht wird. Die Wahl der zerstörbaren Fasern ermöglicht es, durch deren Durchmesser, die Länge sowie durch die Menge der zugesetzten Fasern, die innere Oberfläche der erfindungsgemäßen Katalysatoren in dem notwendigen und angegebenen Bereich zu halten.

Diese neuen Katalysatoren sind vorteilhaft einsetzbar bei Oxidationen von Olefinen wie Propylen oder Isobutylen bzw. dessen Derivat tert.-Butanol zu den entsprechenden Aldehyden, Acrolein und Methacrolein, und deren Weiteroxidation zu den ungesättigten Säuren, Acrylsäure oder Methacrylsäure. Sie sind weiter vor allem verwendbar bei Oxidehydrierungen von Isobuttersäure oder ihren Estern zu Methacrylsäure oder ihren Estern.

Für die hydrierende Behandlung von Schwerölen sind aus der DE-A 18 03 773 und der EP-A 237 240 geformte Katalysatoren, die aus Trägern und aktiven Katalysatorkomponenten bestehen, bekannt, bei deren Konfektionierung in Mengen über 10 Gew.-% bis ca. 20 Gew.-% bezogen auf die eingesetzte Katalysatormasse, zugesezte organische Textilfasern bzw. Kohlenstoffasern bei Temperaturen über 400 Grad C, wie z.B. bei Temperaturen von 400 bis 1 600 Crad C oder bei Temperaturen von 500 bis 800 Grad C durch Calcinieren in Luft entfernt werden, wodurch Katalysatoren mit vielen Kanälen entstehen, und die große innere Oberflächen, d.h. solche von 100 bis 400 $m^2$/g haben.

Katalysatoren dieser bekannten Art sind für die Herstellung ungesättigter $C_3$- und $C_4$-Aldehyde und Carbonsäuren, vor allem der Methacrylsäure oder auch ihrer Ester in Oxidations- bzw. Oxidehydrierungsverfahren nicht brauchbar.

Die erfindungsgemäßen Katalysatoren und die erfindungsgemäß verwendeten Katalysatoren enthalten als aktives Material Stoffe, die Molybdän, Phosphor, Vanadium und gegebenenfalls weitere Elemente, insbesondere Metalle, als einen oxidischen Verband enthalten, und die der chemischen Zusammensetzung $H_aM_bP_cMo_dV_eO_f$ mit den oben angegebenen Bedeutungen für M, a, b, c, d, e und f entsprechen. Diese katalytisch wirksamen Stoffe sind, wie im Stand der Technik beschrieben, vor allem Phosphor-Molybdän-Heteropolysäuren, insbesondere deren Vanadiumderivate, die sich durch die chemische Formel $H_{3+x}PMo_{12-x}V_xO_{40}$ mit x = 0,1,2,3 beschreiben lassen oder die $H_8PMo_{10}VO_{39}$-Heteropolysäure oder deren Anhydrid $PMo_{10}VO_{35}$, sowie Metallderivate dieser Heteropolysäuren bzw. ihrer Metallderivate, wie dies aus der EP-B 0 255 639 bekannt ist. Für die Oxidehydrierung von Isobuttersäure oder deren Ester beispielsweise, haben sich besonders Cu-haltige oder Cu- und Cs-haltige Mo-, P- und V-haltige Katalysatoren, die vorteilhaft aus den beschriebenen Heteropolysäuren hergestellt werden, bewährt.

Zur Herstellung der erfindungsgemäß verbesserten Katalysatorteilchen werden die durch Calcinierung im Temperaturbereich von 100 bis 380 Grad C verbrennbaren und dabei Kanäle hinterlassenden Fasern, mit dem katalytisch aktiven Material, das zunächst auch noch in z.B. wäßriger Lösung vorliegen kann, und gegebenenfalls weiteren Zusatzstoffen, wie bekannten anorganischen und/oder organischen Hilfsmitteln, z.B. Füllstofffen, Bindemitteln oder Gleitmitteln, oder den aus der DE-A37 10 784 bekannten wasserlöslichen Oligomeren oder Polymeren als Zusatzstoffen, vor der Verfestigung zu diskreten Teilchen, vorteilhaft in Gegenwart von Wasser, gut vermischt. Diese faserhaltigen, gegebenenfalls breiigen Mischungen werden dann beispielsweise durch Komprimierung zu Pellets oder durch Extrusion zu stabartigen Teilchen in die gewünschte Form mit den gewünschten Abmessungen gebracht. Die faserhaltigen Mischungen lassen sich aber auch in die Hohlräume von geformten und insbesondere von weitporigen inerten Trägern, wie dies z.B. der kommerziell von Norton vertriebene kugelförmige Silicium-Carbid-Träger Crystolon® C, SC 5232, mit 40 - 45 % Porosität, einer inneren Oberfläche von 0,01 - 0,3 m²/g und einer chemischen Zusammensetzung aus 65,8 Gew.-% SiC und 28,5 Gew.-% $SiO_2$ ist, einbringen, wodurch ein besonderer Arbeitsschritt zur Teilchenformung entfällt.

Für die Erzeugung der Kanäle in den erfindungsgemäßen Katalysatoren sind sowohl natürliche als auch synthetische organische Fasern (Textilfasern) und Kohlenstoff-Fasern, die ihrerseits durch Verkohlung und Graphitisierung von natürlichen und synthetischen organischen, d.h. aus Kohlenstoffverbindungen bestehenden Fasern, entstehen, brauchbar. Diese werden, bei der Calcinierung im Temperaturbereich von 100 bis 380 Grad C, insbesondere in Gegenwart von Sauerstoff zerstört, und letztlich zu gasförmigen Produkten unter Hinterlassung von Hohlräumen, die praktisch den Dimensionen der vorher vorhandenen Fasern entsprechen, abgebaut. Als Naturfasern können beispielsweise Baumwolle, Wolle und Cellulosederivate und als organische Fasern solche aus Polyamiden (Nylon), Polyester, Polyolefinen oder Acrylnitril verwendet werden.

Die erfindungsgemäß zu verwendenden Fasern haben Durchmesser von 1 bis 100 μm, insbesondere solche von 5 bis 70 μm, vor allem solche von 15 bis 50 μm und Längen, die auch das Mehrfache des Durchmessers des endgültigen Katalysatorteilchens sein können, wie beispielsweise 1 bis 30 mm, vorzugsweise 1 bis 10 mm, insbesondere von 2 bis 8 mm, vor allem von 3 bis 6 mm, und werden in Mengen von 0,5 bis 5 Gew.-%, insbesondere von 1 bis 4 Gew.-% und vor allem von 1,5 bis 3,5 Gew.-% bezogen auf das im Katalysator vorhandene, katalytisch aktive Material, zugesetzt.

Die Katalysator- und Fasern-haltigen geformten Teilchen mit Durchmessern oder Längen von 1 bis 10 mm, vor allem solchen mit 2 bis 8 mm und insbesondere solchen von 3 bis 6 mm werden dann, wenn nötig, noch bei Temperaturen bis 100 Grad C getrocknet und weiter durch Behandlung bei Temperaturen über 100 Grad C, vor allem im Bereich von 200 bis 380 Grad C, insbesondere im Bereich von 250 bis 350 Grad C mit Sauerstoff bzw. Sauerstoff-haltigen Gasen, vor allem in Gegenwart von Luft, einer Calcinierung unterworfen. Diese Behandlung des geformten Katalysatormaterials kann in einer eigens für Calcinierungen bestimmten Apparatur, z.B. einem Drehrohrofen, durchgeführt werden; sie kann aber auch in dem Reaktor, z.B. in einem Rohrbündelreaktor, durchgeführt werden, in dem die katalytischen Oxidationsreaktionen bzw. Oxidehydrierungsreaktionen mit dem erfindungsgemäßen Katalysator ausgeführt werden.

Die Erfindung ist bedeutsam für die Herstellung von selektiveren und aktiveren Oxidationskatalysatoren, die in Gasphasenoxidationsreaktionen im Temperaturbereich von 200 bis 400 Grad C verwendet werden, vor allem für die Oxidation von Olefinen, wie Propylen oder Isobutylen zu Acrolein oder Methacrolein, oder für deren Weiteroxidation zu Acrylsäure oder Methacrylsäure. Besondere Bedeutung haben Katalysatoren nach der vorliegenden Erfindung für die oxidative Dehydrierung von Isobuttersäure oder ihrer niederen Ester zu Methacrylsäure oder ihrer niederen Ester, die vorteilhaft an Katalysatoren auf der Basis von Heteropolysäuren des Molybdäns, auch von Salzen dieser Heteropolysäuren durchgeführt wird. Die Oxidehydrierung beispielsweise von Isobuttersäure, aber auch die ihrer niederen Alkylester, wird an diesen Katalysatoren im Temperaturbereich von etwa 250 bis 400 Grad C in Gegenwart von 1 bis 4 Mol Sauerstoff pro Mol Isobuttersäure durchgeführt, wobei weitere Inertgase wie u.a. Stickstoff oder Wasserdampf oder $CO_2$ zugegen sein können.

In den folgenden Beispielen werden erfindungsgemäße, verbesserte, d.h. aktivere und vor allem selektivere P-Mo-V-haltige Heteropolysäure-Katalysatoren bei der Oxidehydrierung von Isobuttersäure zu Methacrylsäure im Vergleich zu solchen des Standes der Technik beschrieben. Die Verweilzeit und damit der Kehrwert der Katalysatorbelastung wird durch den Quotienten $\frac{W}{F}$ mit der Dimension Stunde (h) quantifiziert, wobei W das Gewicht der katalytisch aktiven Masse und F das Gewicht der pro Stunde umzusetzenden Wertsubstanz, wie hier der Isobuttersäure, in gleichen Gewichtseinheiten, bedeutet.

Beispiel 1:

Ein Katalysator aus 70 Gew.-% $Cu_{0,2}H_{3,6}PMo_{11}VO_{40}$ und 29 Gew.-% Kieselgur und 1 Gew.-% Aerosil® als Träger, wurde nach Zusatz von 25 Gew.-% Avicel® als Presshilfsmittel und verschiedenen Fasern aus Polypropylen bzw. Viskose (je 0,5 Gew.-% bez. auf Katalysatormasse) extrudiert. Der so dargestellte und 5 Stunden bei 300 Grad C luftgetemperte Katalysator wurde bei der Oxidehydrierung von Isobuttersäure in der Gasphase eingesetzt. Die Komponenten des über diesen Katalysator geleiteten gasförmigen Gemisches aus Isobuttersäure, Sauerstoff und Stickstoff standen im molaren Verhältnis von 1 : 1,5 : 7,71 zueinander. Die Temperatur des Katalysators betrug 360 Grad C und W/F = 0,8 (h).

| Katalysator *) | Isobuttersäure-Umsatz (%) | Selektivitäten (in %) | | | | | | RZA an MMA (g/h) |
|---|---|---|---|---|---|---|---|---|
| | | MMA | CO2 | CO | Propen | Aceton | Essigs. | |
| ohne Fasern [1] | 89,3 | 74,2 | 6,0 | 5,8 | 2,7 | 8,8 | 2,6 | 344 |
| mit PP-Fasern [2] | 82,4 | 76,9 | 4,3 | 4,4 | 4,1 | 9,7 | 0,7 | 377 |
| mit Viskose-Fasern 3) | 80,9 | 75,5 | 4,9 | 5,3 | 2,9 | 10,5 | 0,9 | 299 |

MMA = Methacrylsäure

*) Innere Oberfläche
1) 1,9 m²/g
2) 2,0 m²/g
3) 2,1 m²/g

Beispiel 2

Der Katalysator wurde analog zu Beispiel 1 hergestellt und mit Fasermaterial extrudiert. Der so dargestellte und bei 300 Grad C 5 Stunden in Sauerstoffatmosphäre getemperte Katalysator wurde bei Oxidehydrierung von Isobuttersäure in der Gasphase eingesetzt. Die Komponenten des über diesen Katalysator geleiteten gasförmigen Gemisches aus Isobuttersäure, Sauerstoff und Stickstoff standen im molaren Verhältnis von 1 : 1,3 : 22 zueinander. Die Temperatur des Katalysators wurde so gewählt, daß ein konstanter Umsatz von ca. 86 % ± 1 erzielt wurde. W/F betrug 1,0 (h).

| Katalysator | Temp. | Isobuttersäure Umsatz (%) | Selektivitäten (in %) | | | | | | RZA an MMA (g/h) |
|---|---|---|---|---|---|---|---|---|---|
| | | | MMA | CO2 | CO | Propen | Aceton | Essigs. | |
| ohne Fasern | 330 | 86,8 | 80,4 | 3,8 | 4,5 | 1,8 | 8,9 | 0,5 | 290 |
| PP-Fasern | 350 | 85,6 | 83,6 | 3,2 | 3,9 | 2,3 | 6,4 | 0,6 | 341 |
| Viskose-Fas. | 350 | 86,6 | 81,9 | 3,5 | 3,9 | 2,3 | 8,4 | 0,0 | 277 |

Beispiel 3

Der Katalysator wurde analog Beispiel 1 hergestellt und mit Fasermaterial extrudiert. Der so dargestellte und in Sauerstoffatmosphäre 5 Stunden bei 300 Grad C calcinierte Katalysator wurde bei der Oxidehydrierung von Isobuttersäure in der Gasphase eingesetzt. Die Komponenten des über diesen Katalysator geleiteten gasförmigen Gemisches aus Isobuttersäure, Wasser, Sauerstoff und Stickstoff standen im molaren Verhältnis von 1 : 2 : 1 : 16 zueinander. Die Temperatur des Katalysators wurde so gewählt, daß ein konstanter Umsatz von ca. 85 ± 2 % erzielt wurde. W/F betrug 2,0 (h).

| Katalysator | Temp. | Isobuttersäure- Umsatz (%) | Selektivitäten (in %) | | | | |
|---|---|---|---|---|---|---|---|
| | | | MMA | CO2 | CO | Propen | Aceton |
| ohne Fasern | 318 | 86,6 | 73,9 | 4,9 | 5,8 | 2,4 | 11,4 |
| PP-Fasern | 323 | 84,6 | 76,9 | 4,0 | 4,7 | 3,4 | 10,5 |
| Viskose-Fas | 325 | 83,0 | 75,3 | 4,5 | 5,5 | 2,8 | 11 |

Beispiel 4

Ein Katalysator aus 70 Gew.-% $Cu_{0,2}H_{3,6}PMo_{11}VO_{40}$ und 30 Gew.-% Kieselgur und Aerosil® (Aerosil® 200 bzw. OX50, Verhältnis 29 : 1 bzw. 5 : 1) als Träger, wurde mit Viskosefasern (0,5 Gew.-% bez. auf Katalysatormasse) bzw. mit 2 Gew.-% Graphit als Zuschlagstoff granuliert. Der so dargestellte und 5 Stunden bei 300 Grad C in Luft getemperte Katalysator wurde bei der Oxidehydrierung von Isobuttersäure in der Gasphase eingesetzt. Die Komponenten des über diesen Katalysator geleiteten gasförmigen Gemisches aus Isobuttersäure, Sauerstoff und Stickstoff standen im molaren Verhältnis von 1 : 1,5 : 7,71 zueinander. Die Temperatur des Katalysators betrug 360 Grad C und W/F = 0,8 (h).

6

| Katalysator *) | Isobuttersäure-Umsatz (%) | Selektivitäten (in %) | | | | | | RZA an MMA (g/h) |
|---|---|---|---|---|---|---|---|---|
| | | MMA | CO2 | CO | Propen | Aceton | Essigs. | |
| Kieselgur/Aerosil®200 (29 : 1) Viskosefaser 1) | 89,3 | 77,6 | 5,0 | 5,2 | 3,5 | 7,2 | 1,6 | 499 |
| Kieselgur/Aerosil®200 (29 : 1) Graphit ohne Fasern 2) | 91,2 | 74,0 | 5,4 | 6,4 | 3,7 | 8,4 | 2,1 | 420 |
| Kieselgur/Aerosil®OX50 (29 : 1) ohne Fasern 3) | 88,2 | 76,7 | 4,7 | 5,4 | 3,3 | 8,5 | 1,4 | 454 |
| Kieselgur/Aerosil®200 (5 : 1) ohne Fasern 4) | 89,2 | 76,2 | 4,8 | 5,1 | 3,9 | 8,6 | 1,4 | 423 |

*) Innere Oberfläche:
1) 2,0 m²/g
2) 1,9 m²/g
3) 1,7 m²/g
4) 2,1 m²/g

**Patentansprüche**

1. Gasphasenoxidationskatalysatoren, bestehend aus dem katalytisch aktiven Material, das Molybdän, Phosphor und Vanadium als wesentliche Elemente in oxidischer Form enthält, und gegebenenfalls noch aus weitgehend chemisch inerten anorganischen Bindemitteln, Verdünnungsmitteln bzw. Trägern, sowie im wesentlichen gleichförmig verteilten Kanälen,
    dadurch gekennzeichnet,
daß bei der Herstellung der Katalysatoren mit Stoffen der chemischen Zusammensetzung $H_aM_bP_cMo_dV_eO_f$, wobei bedeuten

M mindestens ein Metallion von Li, Na, K, Rb, Cs, Mg, Ca, Zn, Al, Ce, Ti, Zr, Sn, Sb, As, Bi, Cr, Mn, Fe, Co, Ni oder Cu

a = 0 bis 8

b = 0 bis 6

c = 1 bis 2

d = 9 bis 12

e = 0,2 bis 3

f = Atomzahl, die sich zur stöchiometrischen Absättigung der übrigen Komponenten aus deren Wertigkeiten und Anteilen ergibt,

als dem katalytisch aktivierten Material oder als dessen Vorläufer und gegebenenfalls aus den inerten Zusatzstoffen, vor der Formgebung noch 0,5 bis 5 Gew.-%, bezogen auf das katalytisch wirkende Material, Kohlenstoff- bzw. organische Fasern mit einem Durchmesser von 1 bis 100 µm zugemischt werden, und die nach der Formgebung zur Bildung der Kanäle, durch Calcinieren der Formlinge bei Temperaturen im Bereich von 100 bis 380 Grad C in Gegenwart von Sauerstoff wieder daraus entfernt werden.

**2.** Oxidationskatalysatoren nach Anspruch 1, dadurch gekennzeichnet, daß die Kanäle durch Herausbrennen von Kohlenstoff-Fasern hergestellt werden.

**3.** Oxidationskatalysatoren nach Anspruch 1, dadurch gekennzeichnet, daß die Kanäle durch Herausbrennen von organischen Fasern hergestellt werden.

**4.** Oxidationskatalysatoren nach den Ansprüchen 1 bis 3, dadurch gekennzeichnet, daß die innere Oberfläche so hergestellter Katalysatoren unter 5 m²/g, vor allem im Bereich von 0,1 bis 5 m²/g, insbesondere im Bereich von 0,5 bis 2,5 m²/g liegt.

**5.** Verwendung von Gasphasenoxidationskatalysatoren bei der Oxidation von Propylen oder Isobutylen bzw. tert.-Butanol zu Acrolein oder Methacrolein und/oder deren Weiteroxidation bzw. bei deren Oxidation zu Acrylsäure oder Methacrylsäure im Temperaturbereich von 200 bis 400 Grad C, dadurch gekennzeichnet, daß als Katalysatoren solche nach den Ansprüchen 1 bis 4 hergestellte verwendet werden.

**6.** Verwendung von Gasphasenoxidationskatalysatoren bei der oxidativen Dehydrierung von Isobuttersäure oder ihrer niederen Estern zu Methacrylsäure oder ihrer niederen Estern bei Temperaturen von 250 bis 400 Grad C, dadurch gekennzeichnet, daß als Katalysatoren solche nach den Ansprüchen 1 bis 4 hergestellte verwendet werden.

## Claims

**1.** Vapour-phase oxidation catalysts consisting of catalytically active material, which contains molybdenum, phosphorus and vanadium as essential elements in oxidised form and, optionally, further largely chemically inert inorganic binding agents, diluents or carriers, as well as largely uniformly distributed channels, characterised in that, when preparing catalysts with substances of the chemical composition $H_aM_bO_cMo_dV_eO_f$, in which

M is at least one metal ion of Li, Na, K, Rb, Cs, Mg, Ca, Zn, Al, Ce, Ti, Zr, Sn, Sb, As, Bi, Cr, Mn, Fe, Co, Ni or Cu
a = 0 to 8
b = 0 to 6
c = 1 to 2
d = 9 to 12
e = 0.2 to 3
f = atom number resulting from the values and amounts of the other components for the stoichiometric saturation thereof,

which substances act as the catalytically active material or the precursor thereof and optionally consist of the inert additives, carbon or organic fibres with a diameter of 1 to 100 μm are also added, before shaping, in an amount of 0.5 to 5 wt.% based on the catalytically active material, and which fibres are removed again from the material after shaping in order to produce channels, by calcinating the shaped pieces at temperatures within the range of 100 to 380°C in the presence of oxygen.

**2.** Oxidation catalysts according to claim 1, characterised in that the channels are produced by the burning-out of carbon fibres.

**3.** Oxidation catalysts according to claim 1, characterised in that the channels are produced by the burning-out of organic fibres

**4.** Oxidation catalysts according to claims 1 to 3, characterised in that the internal surface of the catalysts thus produced is less than 5 m²/g, particularly within the range of 0.1 to 5 m²/g, more particularly within the range of 0.5 to 2.5 m²/g.

**5.** Use of vapour-phase oxidation catalysts in the oxidation of propylene or isobutylene or tert.-butanol to form acrolein or methacrolein and/or in the further oxidation thereof or in the oxidation thereof to form acrylic acid or methacrylic acid within the temperature range of 200 to 400°C, characterised in that those catalysts are used which have been prepared according to claims 1 to 4.

**6.** Use of vapour-phase oxidation catalysts in the oxidative dehydrogenation of isobutyric acid or the lower esters thereof to form methacrylic acid or the lower esters thereof at temperatures of between 250 to 400°C, characterised in that those catalysts are used which have been prepared according to claims 1 to 4.

**Revendications**

1. Catalyseurs d'oxydation en phase gazeuse, constitués de la matière catalytique active, qui contient du molybdène, du phosphore et du vanadium sous forme d'oxydes comme éléments essentiels, et éventuellement encore de liants inorganiques chimiquement inertes dans une large mesure, d'agents d'allongement ou de supports, ainsi que de canaux régulièrement répartis pour l'essentiel, caractérisés en ce que, dans la préparation des catalyseurs, on mélange à des matières de composition chimique $H_aM_bP_cMo_dV_eO_f$, dans laquelle

   M      représente au moins un ion métallique de Li, Na, K, Rb, Cs, Mg, Ca, Zn, Al, Ce, Ti, Zr, Sn, Sb, As, Bi, Cr, Mn, Fe, Co, Ni ou Cu

   a =     0 à 8

   b =     0 à 6

   c =     1 à 2

   d =     9 à 12

   e =     0,2 à 3

   f=      nombre atomique permettant d'obtenir une compensation stoechiométrique des autres composants en fonction de leurs valences et proportions,

   en tant que matière catalytiquement active ou en tant que son précurseur, et éventuellement à partir des adjuvants inertes, avant le façonnage, encore 0,5 à 5% en poids, par rapport à la matière catalytiquement active, de fibres de carbone ou de fibres organiques de diamètres compris entre 1 et 100 μm, que l'on élimine après le façonnage pour former les canaux, par calcination des produits moulés à des températures comprises entre 100 et 380°C en présence d'oxygène.

2. Catalyseurs d'oxydation selon la revendication 1, caractérisés en ce que l'on forme les canaux par calcination de fibres de carbone dans la matière.

3. Catalyseurs d'oxydation selon la revendication 1, caractérisés en ce que l'on forme les canaux par calcination de fibres organiques dans la matière.

4. Catalyseurs d'oxydation selon l'une quelconque des revendications 1 à 3, caractérisés en ce que la surface interne de catalyseurs ainsi préparés est inférieure à 5 $m^2$/g, de préférence comprise entre 0,1 et 5 $m^2$/g, en particulier entre 0,5 et 2,5 $m^2$/g

5. Utilisation de catalyseurs d'oxydation en phase gazeuse dans l'oxydation de propylène ou d'isobutylène ou de tert.-butanol en acroléine ou en méthacroléine et/ou dans leur oxydation subséquente ou dans leur oxydation en acide acrylique ou en acide méthacrylique à des températures de 200 à 400°C, caractérisée en ce que l'on utilise comme catalyseurs des catalyseurs préparés selon l'une quelconque des revendications 1 à 4.

6. Utilisation de catalyseurs d'oxydation en phase gazeuse dans la déshydrogénation oxydante d'acide isobutyrique ou de ses esters inférieurs en acide méthacrylique ou en ses esters inférieurs à des températures de 250 à 400°C, caractérisée en ce que l'on utilise comme catalyseurs, les catalyseurs préparés selon les revendications 1 à 4.